# EUROPEAN PATENT SPECIFICATION

(11) **EP 2 442 847 B1**
(45) Date of publication and mention of the grant of the patent: **28.08.2013**
(21) Application number: 10763389.3
(22) Date of filing: 15.06.2010
(51) Int. Cl.: A61M 1/16

(54) **DIALYSIS MACHINE CALIBRATION**
DIALYSEMASCHINENKALIBRIERUNG
ÉTALONNAGE D'APPAREIL DE DIALYSE

(30) Priority: 15.06.2009 GB 0910247
(43) Date of publication of application: 25.04.2012
(73) Proprietor: Quanta Fluid Solutions Ltd, Warwickshire B49 6EU (GB)
(72) Inventor: WALLACE, Mark, South Staffordshire DY7 6EL (GB); HIGGIT, Ben, Birmingham B47 5RD (GB); HEYES, Keith, Worcestershire B45 8NR (GB)
(74) Representative: Croston, David
(86) International application number: PCT/GB2010/001160
(87) International publication number: WO 2010/146342

(56) References cited:
- EP-A1- 0 623 357
- WO-A2-2005/089832
- WO-A2-2008/106191
- US-B1- 6 228 047

## Description

The present invention relates to dialysis machines and in particular, but not exclusively, to a disposable cartridge for use in hemodialysis machine.

Dialysis is a treatment which replaces the renal function of removing excess fluid and waste products, such as potassium and urea, from blood. The treatment is either employed when renal function has deteriorated to an extent that uremic syndrome becomes a threat to the body's physiology (acute renal failure) or, when a longstanding renal condition impairs the performance of the kidneys (chronic renal failure).

There are two major types of dialysis, namely hemodialysis and peritoneal dialysis.

In peritoneal dialysis treatment, a dialysate solution is run through a tube into the peritoneal cavity. The fluid is left in the cavity for a period of time in order to absorb the waste products, and is subsequently removed through the tube for disposal.

It is common for patients in the early stages of treatment for a longstanding renal condition to be treated by peritoneal dialysis before progressing to hemodialysis at a later stage.

In hemodialysis, the patient's blood is removed from the body by an arterial line, is treated by the dialysis machine, and is then returned to the body by a venous line. The machine passes the blood through a dialyser containing tubes formed from a semi-permeable membrane. On the exterior of the semi-permeable membrane is a dialysate solution. The semi-permeable membrane filters the waste products and excess fluid from the blood into the dialysate solution. The membrane allows the waste and a controlled volume of fluid to permeate into the dialysate whilst preventing the loss of larger -more desirable molecules, like blood cells and certain proteins and polypeptides.

The action of dialysis across the membrane is achieved primarily by a combination of diffusion (the migration of molecules by random motion from a region of higher concentration to a region of lower concentration), and convection (solute movement that results from bulk movement of solvent, usually in response to differences in hydrostatic pressure).

Fluid removal (otherwise known as ultrafiltration) is achieved by altering the hydrostatic pressure of the dialysate side of the membrane, causing free water to move across the membrane along the pressure gradient.

The correction of uremic acidosis of the blood is achieved by use of a bicarbonate buffer. The bicarbonate buffer also allows the correction of the blood bicarbonate level.

The dialysis solution consists of a sterilized solution of mineral ions. These ions are contained within an acid buffer which is mixed with water and bicarbonate base prior to delivery to the dialyser. The water used is cleaned to a sufficient degree that it is suitable for use as a base for trans-membrane ion transfer with the blood (hereinafter sterile water), this may for example be achieved by known methods including reverse osmosis, heat treatment, filtration or a combination of such known methods.

Dialysate composition is critical to successful dialysis treatment since the level of dialytic exchange across the membrane, and thus the possibility to restore adequate body electrolytic concentrations and acid-base equilibrium, depends on the composition.

Document WO 2005/089832 shows a system for calibrating the dialysate composition.

The correct composition is accomplished primarily by formulating a dialysate whose constituent concentrations are set to approximate normal values in the body.

However, achieving the correct composition of dialysate requires the accurate control of low volumes of liquid and at present this is achieved by the provision of complex fluid paths, including multiple pumping and valving components on the dialysis machine.

This presents the disadvantage of a complex and costly dialysis machine which is at increased risk of failure by virtue of its complexity. Increased maintenance is also a problem since it is essential to minimise machine downtime in order to most efficiently treat the patient. In addition the complexity of these systems within the machine results in overly large machines that cannot be easily transported or kept, in an unobtrusive manner, in the home.

A further problem with known hemodialysis machines is that the blood and dialysate solution lines require careful mounting onto the dialysis machine before the treatment can commence. This presents a risk that the lines are not correctly installed, a risk which is particularly relevant to those patients who dialyse at home.

This method of dialysis also presents an increased risk of cross-infection between patients since the disposable blood and dialysate lines come into contact with the dialysis machine which needs frequent and thorough cleaning between uses.

The problems associated with conventional dialysis equipment are mitigated to some degree by the system disclosed in WO 2006/120415 which discloses a cartridge based system for conducting hemodialysis, however the method and system for mixing the dialysate proposed in this application is complex and costly involving a large cartridge with multiple reservoirs, each having level control and therefore requiring a complex pumping and control system. Both this complexity and this space requirement are undesirable in portable dialysis machines, for example those suitable for home dialysis.

It is an object of the present invention to provide a hemodialysis system which at least mitigates some of the problems described above.

According to a first aspect of the invention there is provided a cartridge for use in a hemodialysis machine, the cartridge comprising:
a dialysate flow path including a dialyser, the dialysate flow path for delivering a flow of dialysate through the dialyser;
a mixing pump defining a chamber having volume variable between a maximum volume and a minimum volume for receiving a predetermined volume of a first dialysate solution base, second dialysate solution base, and a volume of water;
a first dialysate solution base supply conduit having a first positive displacement pump having a first inlet valve and a first outlet valve associated therewith;
a second dialysate solution base supply conduit having a second positive displacement pump having a second inlet valve and a second outlet valve associated therewith;
a first and second fluid conduit associated joining respective first and second positive displacement pumps with the mixing pump; and
a third fluid conduit for connecting the mixing pump outlet to a dialyser filter inlet.

By using this apparatus an accurately mixed dialysate can be produced using a minimum of pump chambers and sensors.

Preferably the cartridge comprises a blood flow path for carrying a volume of blood to be treated in the dialyser.

Preferably the cartridge is disposable.

Preferably said first and second conduits each have a sensor therein to generate a signal indicative of the concentration of said first and second dialysate solution base flowing through said conduits.

Preferably the cartridge further comprises a sensor in the third conduit to generate a signal indicative of the concentration of the fluid flowing therethrough.

In a preferred arrangement the first and second fluid conduits feed directly into the mixing pump chamber. In an alternative preferred arrangement the first and second fluid conduits feed into a water conduit immediately upstream of the chamber of the mixing pump.

Preferably the cartridge further comprises:
a diverter valve in the third conduit, downstream of the sensor therein, having a first position in which fluid passing therethrough is directed to the dialyser and a second position in which fluid passing therethrough is diverted to a drain.

The diverter valve enables the flow path out of the mixing chamber to be directed to either a dialyser or to the drain, thus avoiding flowing out of specification dialysate through the dialyser.

Preferably the first inlet valve and first outlet valve, and the second inlet valve and second outlet valve comprise first and second anti siphon check valves, each having three ports and two flow paths therethrough, one connecting the dialysate solution base supply conduit to the associated positive displacement and the second connecting the associated positive displacement pump to the mixing pump chamber.

According to a second aspect of the invention there is provided a method of calibrating a dialysate mixing pump for mixing a tri-mix dialysate comprising a mixture of first dialysate base solution, second dialysate base solution and water in specific ratios, said method comprising the steps of:
a) predicting the required volume of a first dialysate solution base needed to create a dialysate having a required concentration of said first dialysate solution base;
b) operating a first dialysate solution base pump to add said predicted volume of first dialysate solution base to a volume of water in a mixing pump and mixing said first dialysate solution base with said water therein to form a first mixture;
c) expelling said first mixture from the mixing pump and measuring a parameter indicative of the concentration of said first mixture;
d) modifying the first dialysate solution base pump controls to increase or decrease the amount of first dialysate solution base added to said mixing pump dependant on the measured a parameter;
e) predicting the required volume of a second dialysate solution base needed to create a dialysate having a required concentration of said second dialysate solution base;
f) operating a second dialysate solution base pump to add said predicted volume of second dialysate solution base to a volume of water in a mixing pump and mixing said second dialysate solution base with said water therein to form a second mixture;
g) expelling said second mixture from the mixing pump and measuring a parameter indicative of the concentration of said second mixture;
h) modifying the second dialysate solution base pump controls to increase or decrease the amount of second dialysate solution base added to said mixing pump dependant said parameter;
i) operating the first and second dialysate solution base pumps to add the modified volume of first and second dialysate solution bases to a volume of water in the mixing pump chamber to achieve a required tri-mix of first dialysate base solution, second dialysate base solution and water.

By this method the apparatus having a minimum number of mixing chambers and sensors can be calibrated to produce an accurately mixed dialysate.

Preferably the method further comprises the step of measuring a parameter indicative of the concentration of the first dialysate solution base and using said measurement in the prediction of the required volume of the first dialysate solution base needed.

Preferably the method further comprises the step of measuring a parameter indicative of the concentration of the second dialysate solution base and using said measurement in the prediction of the required volume of the second dialysate solution base needed.

Preferably after step h) the method further comprises the step of repeating steps e) to h) until the concentration of the first mixture is equal to the required concentration of the first dialysate solution base.

Preferably the method further comprises the step of: once the required concentration is achieved, recording a parameter indicative of the volume of first dialysate solution base added to said mixing pump to create a first mixture having the required concentration.

Preferably after step d) the method further comprises the step of repeating steps a) to d) until the concentration of the second mixture is equal to the required concentration of the second dialysate solution base.

Preferably the method further comprises the step of: once the required concentration is achieved, recording a parameter indicative of the volume of second dialysate solution base added to said mixing pump to create a second mixture having the required concentration.

Preferably the method further comprises: measuring a parameter indicative of the concentration of the tri-mix to verify it has the correct concentration. Preferably the method further comprises the step of: if the measured parameter indicates the tri-mix does not have the correct concentration, repeating the calibration.

Preferably the method further comprises monitoring a parameter indicative of the concentration of the first dialysate solution base.

Preferably the method further comprises the step of: if the measured parameter indicates first dialysate solution base changes concentration beyond a specified range, repeating the steps of claim 8.

Preferably the method further comprises the step of: monitoring a parameter indicative of the concentration of the second dialysate solution base. Preferably the method further comprises the step of: if the measured parameter indicates second dialysate solution base changes concentration beyond a specified range, repeating the calibration.

Preferably the method further comprises: if the mixture of the tri mix dialysate is outside of its specified ratios, diverting the flow of dialysate to drain.

Preferably the method further comprises: if the mixture of the tri mix dialysate is within its specified ratios, diverting the flow of dialysate to a dialyser.

The invention will now be described, by way of example only, and with reference to the following drawings, in which:
Figure 1 is an isometric view of the dialysis machine for use with the cartridge of the current invention,
Figure 2 is an isometric view of the engine portion of the machine of Figure 1,
Figure 3 is a schematic diagram of the dialysate flow path for use on a cartridge of the invention; and
Figure 4 is a schematic representation of a dialysate solution base delivery system according to the present invention.

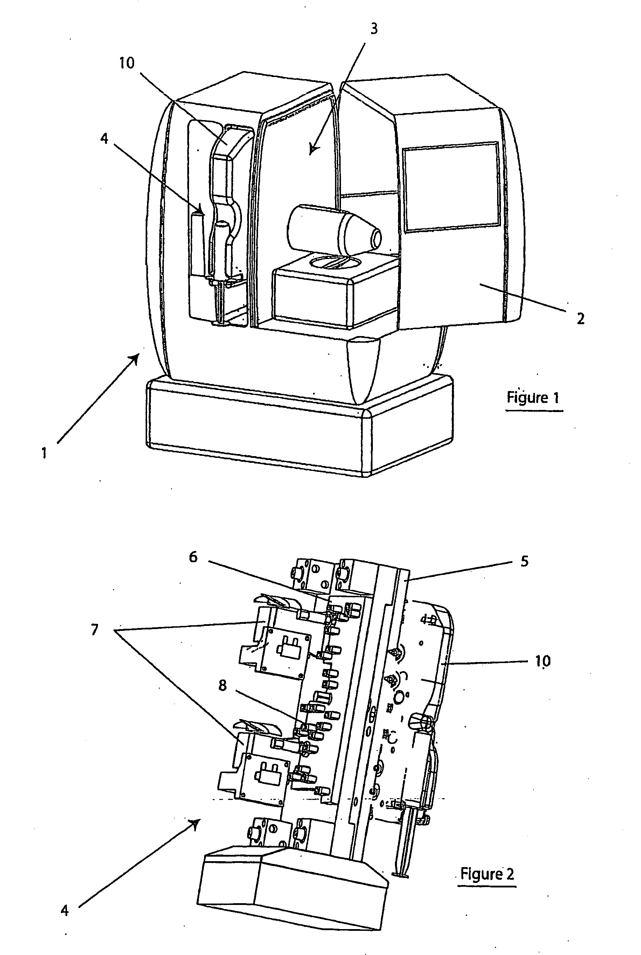

In Figure 1 a dialysis machine 1 is shown having a cover 2 which opens to reveal a storage compartment 3. The machine has an engine section 4 which receives a dialysis cartridge 10.

Referring now to Figure 2, the engine section 4 is shown in further detail to include first and second platens 5, 6 which close upon insertion of the cartridge 10 into the machine to retain the cartridge in position in use. The engine 4 has pneumatic actuators 7 and sensors (indicated generally at 8 in Figure 2) arranged on the second platen to control operation of the cartridge 10 as will be described in further detail shortly.

In general the dialysate cartridge may have all the fluid flow paths and functions for the supply of dialysate and blood to a dialyser incorporated therein, or may purely be a cartridge for the preparation of dialysate. The cartridge comprises a moulded plastics cartridge having fluid flow paths defined in the surface thereof. At least one surface of the cartridge is covered by a flexible membrane, formed from a deformable plastics material, enclosing the fluid flow paths. The cartridge has a number of inlets for clean water, first dialysate solution base and second dialysate solution base, and an outlet to drain. The cartridge may also have an outlet for the mixed tri-mix dialysate from which it can be supplied to a dialyser, or alternatively the cartridge may contain all the flow paths necessary for dialysate and blood, and a dialyser filter, onboard such that the entire dialysis process can take place on the cartridge. Further details of how such a cartridge may be constructed can be found in WO 2006/120415.

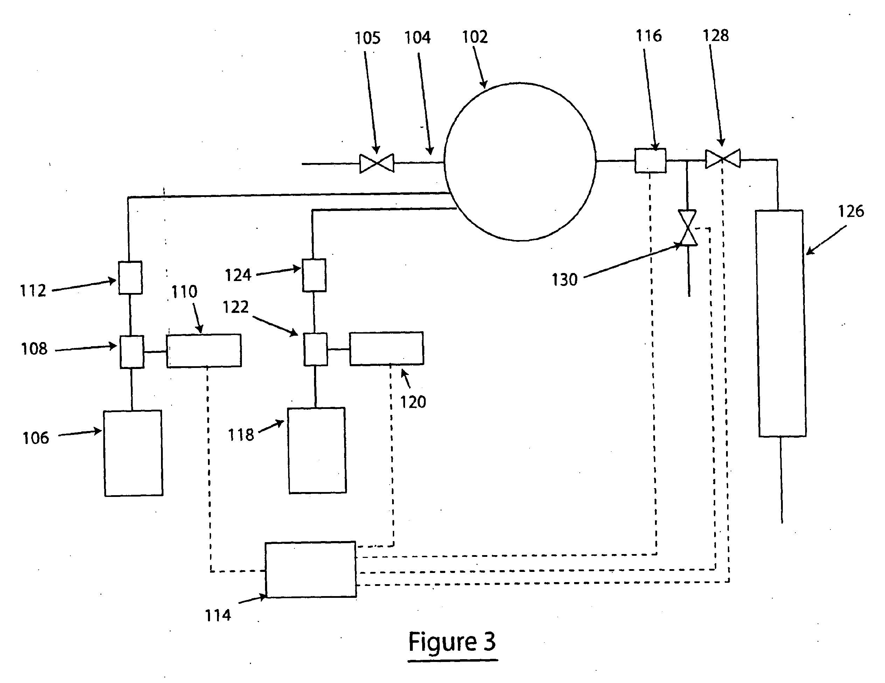

Referring to Figure 3 a schematic diagram is shown of a dialysate preparation flow path suitable for incorporation onto a cartridge for use in the machine of Figure 1. A mixing pump having a chamber 102 is supplied with a supply of clean water via conduit 104 and valve 105. Prior to supply to the chamber the clean water will have undergone treatment, for example reverse osmosis, to clean it and remove any bacteria to a level acceptable for use in a hemodialysis process. A saturated solution of bicarbonate 106 is prepared remotely from the cartridge 100 and fed to a three way valve 108 of a bicarbonate pump 110. A controller 114, having been inputted, either manually or electronically, with a required concentration of bicarbonate in the tri mix, makes an initial calculation of the volume of bicarbonate to be added to the chamber 102 of the mixing pump of a known volume, based on the temperature and operates the bicarbonate pump 110 to add that volume to the chamber 102, via the three way valve 108. The bicarbonate is added either before and/or simultaneously to the water entering the chamber 102 of the mixing pump. Fluid turbulence within the chamber 102 of the mixing pump, both as water is drawn in, and as the mixture is expelled causes the bicarbonate and water to mix thoroughly. As the concentration of the saturated bicarbonate solution can change with temperature and conditions, and as there may be manufacturing tolerance differences in the exact volume of the chamber 102 from one cartridge 100 to another it is virtually impossible to get the exact mixture required, purely by predicting the required volume of bicarbonate needed to be added by the bicarbonate pump 110.

A first conductivity sensor 112 measures the conductivity of the saturated bicarbonate solution to create a signal indicative of its concentration, and feeds this signal back to the controller 114. The controller 114 can then increase or decrease the amount of bicarbonate added by the bicarbonate pump 110 to compensate for any variance between the predicted concentration of the saturated bicarbonate solution and the actual concentration of the bicarbonate solution.

A second conductivity sensor 116 is placed downstream of the pump chamber 102 and measures the conductivity of the fluid exiting the mixing pump chamber 102 to create a signal indicative of the concentration of the mixture being pumped from the chamber 102 and feed it back to the controller 114. The controller 114 can then increase or decrease the amount of bicarbonate added by the bicarbonate pump 110 to achieve the required concentration of bicarbonate in the bicarbonate water mixture generated in the mixing pump.

The process is repeated until the required concentration of bicarbonate and water is achieved. When this is achieved, control parameters for controlling the bicarbonate pump 110 are stored in the controller 114.

The process is then repeated but with acid solution from a supply 118 via an acid solution pump 120 and associated dual check valve 122 and conductivity sensor 124 until the required concentration of acid solution and water has been established. Again signals are fed back to the controller 114 indicative of the concentration of the acid solution being added to the mixing pump chamber 102 and indicative of the concentration of water/acid solution mixture generated in the mixing pump chamber 102, the controller modifying the control of the acid solution pump 120 in response to these signals to achieve the required mixture of acid solution and water. When the required concentration of acid solution and water is achieved, control parameters for controlling the acid solution pump 120 are stored in the controller.

Once the pump control parameters for the bicarbonate pump 110 and the acid solution pump 120 have been independently established, both bicarbonate pump 110 and acid pump 120 are operated simultaneously by the controller to add bicarbonate solution and acid solution respectively to the mixing pump chamber 102 so as to mix with water therein. The tri-mixture dialysate of bicarbonate solution, acid solution and water is then expelled from the chamber 102 and its conductivity is measured by conductivity sensor 116 to verify that it is of the correct concentration. The tri-mixture dialysate can then be passed through a dialyser 126, which may be a part of the cartridge, attached thereto or remote therefrom, and used to dialyze blood. Prior to use, i.e. during the calibration routine, the fluid emitting from the mixing pump chamber is drained. The drain path may flow through the dialyser, i.e. the same path as used in treatment, or alternatively a separate drain path may be provided leading from the conduit joining the mixing pump chamber 102 and the dialyser 126 to a drain point. In this arrangement, valves 128, 130 control the flow of the dialysate to either pass through the dialyser or to flow directly to the drain. In use, if, during the dialysis process, the conductivity sensor 116 detects that the concentration of the tri-mix dialysate has gone out of specification the controller 114 actuates valves 128, 130 to divert the flow of the dialysate to the drain, bypassing the filter, and the calibration process is repeated. Once the pumps and control are again producing dialysate within specification the controller 114 actuates valves 128, 130 again such that the dialysate flows through the dialyser and the process continues.

In addition to the functions described above the sensors 112, 122 can be used as safety sensors and will detect a change of fluid from dialysate solution base to air and thus can detect if the source of dialysate solution base 106, 118 runs out or if air becomes drawn into the system at that point. The flow can then be stopped accordingly to prevent air from entering the dialyser filter 126.

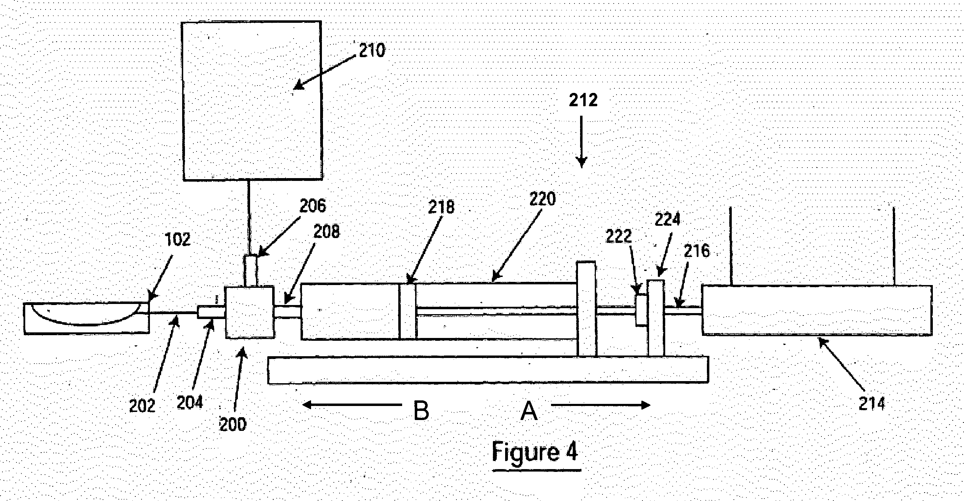

Referring now to Figure 4, the positive pump and three-way valve on the current invention are shown schematically in further detail. The three-way valve is indicated generally at 200.

The mixing pump chamber 102 is connected via a fluid line 202 to an output 204 of the three-way valve 106. The three-way valve also has a reservoir inlet 206 and a pump inlet 208. The reservoir inlet 206 is connected to a reservoir 210 containing the acid or bicarbonate solution. The reservoir 210 is provided on the dialysis machine, or attached thereto, and does not form part of the cartridge itself. The positive displacement pump is indicated generally at 212. The positive displacement pump includes a pneumatic cylinder 214 which drives a piston arm 216 in a reciprocating manner. At the opposite end of the piston arm 216 to the piston cylinder 214 is a plunger 218 which acts within a pump chamber 220 integral within the cartridge.

On the return stroke indicated at A in Figure 4, the plunger 218 is moved within the chamber 220 to draw in to the chamber 220 a measured volume of fluid from the bicarbonate/acid solution reservoir 210. This transfer of fluid is achieved by the closure of the three-way valve output 204, with the reservoir inlet 206 and pump inlet 208 remaining open. The piston arm 216 is withdrawn in direction A until an abutment 222 provided on the piston arm 216 comes into contact with a moveable end stop 224.

Upon the abutment 222 hitting the moveable end stop 224, the pneumatic cylinder 214 is driven in direction B in order to dispense the dialysate solution from the chamber 220 into the mixing pump chamber 102. This transfer of fluids is achieved by the closure of the reservoir inlet 206, and the opening of the three-way valve output 204. The pneumatic cylinder 214 drives the plunger 218 in direction B until the plunger abuts the extreme left hand end of the chamber 220. Accordingly, by reciprocating the movement of the cylinder piston arm 216 in a known manner, a quantity of bicarbonate/acid solution is repeatedly dispensed into the mixing pump chamber 102. Furthermore, by adjusting the position of the removable end stop 224, the volume of fluid dispensed can be accurately set. The moveable end stop 224 is positioned by a stepper motor or similar accurate positioning drive system.

It will be appreciated that the above description is given by way of example only and it is anticipated that various changes may be made to the specific arrangement of components without departing from the scope of the invention as defined by the claims, for example the pumping arrangement may be different from that described in relation to Figure 4.

## Claims

1. A cartridge (10) for use in a hemodialysis machine (1), the cartridge (10) comprising:
a dialysate flow path, including a dialyser, the dialysate flow path for delivering a flow of dialysate through the dialyser;
a mixing pump defining a chamber (102) having volume variable between a maximum volume and a minimum volume for receiving a predetermined volume of a first dialysate solution base, second dialysate solution base, and a volume of water;
a first dialysate solution base supply conduit having a first positive displacement pump (110, 212) having a first inlet valve (108) and a first outlet valve (108) associated therewith;
a second dialysate solution base supply conduit having a second positive displacement pump (120, 212) having a second inlet valve (122) and a second outlet valve (222) associated therewith;
a first and second fluid conduit associated joining respective first and second positive displacement pumps (110, 120, 212) with the mixing pump (102); and
a third fluid conduit for connecting the mixing pump (102) outlet to a dialyser filter inlet (126).

2. A cartridge (10) according to claim 1 wherein said first and second conduits each have a sensor (112, 124) therein to generate a signal indicative of the concentration of said first and second dialysate solution base flowing through said conduits.

3. A cartridge (10) according to claim 1 or claim 2 further comprising a sensor (116) in the third conduit to generate a signal indicative of the concentration of the fluid flowing therethrough.

4. A cartridge (10) according to any one of claims 1 to 3 wherein the first and second fluid conduits feed directly into the mixing pump chamber (102).

5. A cartridge (10) according to claim 3 further comprising:
a diverter valve (128, 130) in the third conduit, downstream of the sensor (116) therein,
having a first position in which fluid passing therethrough is directed to the dialyser and a second position in which fluid passing therethrough is diverted to a drain.

6. A cartridge (10) according to any preceding claim wherein the first and second fluid conduits feed into a water conduit (104) immediately upstream of the chamber (102) of the mixing pump; or wherein the first inlet valve (108) and first outlet valve (108), and the second inlet valve (122) and second outlet valve (122) comprise first and second anti siphon check valves (200), each having three ports and two flow paths therethrough, one connecting the dialysate solution base supply conduit to the associated positive displacement and the second connecting the associated positive displacement pump (110, 120, 212) to the mixing pump chamber (102).

7. A method of calibrating a dialysate mixing pump (102) for mixing a tri-mix dialysate comprising a mixture of first dialysate base solution, second dialysate base solution and water in specific ratios, said method comprising the steps of:
a) predicting the required volume of a first dialysate solution base needed to created a dialysate having a required concentration of said first dialysate solution base;
b) operating a first dialysate solution base pump (110, 212) to add said predicted volume of first dialysate solution base to a volume of water in a mixing pump (102) and mixing said first dialysate solution base with said water therein to form a first mixture;
c) expelling said first mixture from the mixing pump (102) and measuring a parameter indicative of the concentration of said first mixture;
d) modifying the first dialysate solution base pump (110, 212) controls to increase or decrease the amount of first dialysate solution base added to said mixing pump (102) dependent on the measured a parameter;
e) predicting the required volume of a second dialysate solution base needed to create a dialysate having a required concentration of said second dialysate solution base;
f) operating a second dialysate solution base pump (120, 212) to add said predicted volume of second dialysate solution base to a volume of water in a mixing pump (102) and mixing said second dialysate solution base with said water therein to form a second mixture;
g) expelling said second mixture from the mixing pump (102) and measuring a parameter indicative of the concentration of said second mixture;
h) modifying the second dialysate solution base pump (120, 212) controls to increase or decrease the amount of second dialysate solution base added to said mixing pump (102) dependant said parameter;
i) operating the first (110, 212) and second (120, 212) dialysate solution base pumps to add the modified volume of first and second dialysate solution bases to a volume of water in the mixing pump chamber (102) to achieve a required tri-mix of first dialysate base solution, second dialysate base solution and water.

8. The method according to claim 7 further comprising the step of measuring a parameter indicative of the concentration of the first dialysate solution base and using said measurement in the prediction of the required volume of the first dialysate solution base needed.

9. The method according to claim 7 or claim 8 further comprising the step of measuring a parameter indicative of the concentration of the second dialysate solution base and using said measurement in the prediction of the required volume of the second dialysate solution base needed.

10. The method according to any one of claims 7 to 9 wherein after step h) the method further comprises the steps e) to h) until the concentration of the first mixture is equal to the required concentration of the first dialysate solution base.

11. The method according to claim 10 further comprising the step of:
once the required concentration is achieved, recording a parameter indicative of the volume of first dialysate solution base added to said mixing pump (102) to create a first mixture having the required concentration.

12. The method according to any one of claims 7 to 11 wherein after step d) the method further comprises the step of repeating steps a) to d) until the concentration of the second mixture is equal to the required concentration of the second dialysate solution base.

13. The method according to cliaim 12 further comprising the step of:
once the required concentration is achieved, recording a parameter indicative of the volume of second dialysate solution base added to said mixing pump (102) to create a second mixture having the required concentration.

14. The method according to claim 7 further comprising:
measuring a parameter indicative of the concentration of the tri-mix to verify it has the correct concentration.

15. The method according to claim 14 further comprising:
if the measured parameter indicates the tri-mix does not have the correct concentration, repeating the step of claim 7.

16. The method according to claim 14 or claim 15 comprising:
monitoring a parameter indicative of the concentration of the first dialysate solution base.

17. The method according to claim 14 further comprising:
if the measured parameter indicates first dialysate solution base changes concentration beyond a specified range, repeating the steps of claim 8.

18. The method according to claim 7 further comprising:
monitoring a parameter indicative of the concentration of the second dialysate solution base.

19. The method according to claim 18 further comprising:
if the measured parameter indicates second dialysate solution base changes concentration beyond a specified range, repeating the steps of claim 8.

20. The method according to any one of claims 7 to 19 further comprising:
if the mixture of the tri-mix dialysate is outside of its specified ratios, diverting the flow of dialysate to drain.

21. The method according to any one of claims 7 to 20 further comprising:
if the mixture of the tri-mix dialysate is within its specified ratios, diverting the flow of dialysate to a dialyser (126).

22. The cartridge of anyone of claims 1 to 6, wherein the cartridge (10) includes a blood flow path for carrying a volume of blood to be treated by a dialyser (126), or wherein the cartridge (10) is disposable.

## Patentansprüche

1. Einsatz (10) zum Gebrauch in einer Hämodialyse-Anlage (1), umfassend:
eine Dialysat-Flussbahn einschließlich eines Dialysators, wobei die Dialysat-Flussbahn zum Zuführen eines Dialysatflusses durch den Dialysator dient;
eine eine Kammer (102) definierende Mischpumpe, wobei die Kammer ein zwischen einem Maximalvolumen und einem Minimalvolumen variables Volumen zur Aufnahme eines vorbestimmten Volumens einer ersten Dialysatlösung-Basis, zweiten Dialysatlösung-Basis und eines Volumens von Wasser aufweist;
eine erste Dialysatlösung-Basis-Bereitstellungsleitung mit einer ersten positiven Verdrängungspumpe (110, 212) mit einem dazugehörigen ersten Einlassventil (108) und einem dazugehörigen ersten Auslassventil (108);
eine zweite Dialysatlösung-Basis-Bereitstellungsleitung mit einer zweiten positiven Verdrängungspumpe (120, 212) und einem dazugehörigen zweiten Einlassventil (122) und einem dazugehörigen zweiten Auslassventil (222);
eine zugehörige erste und zweite Fluidleitung, welche jeweilige erste und zweite positive Verdrängungspumpen (110, 120, 212) mit der Mischpumpe (102) verbinden; und
eine dritte Fluidleitung zum Anschließen des Auslasses der Mischpumpe (102) an einen Dialysator-Filtereinlass (126).

2. Einsatz (10) nach Anspruch 1, wobei die ersten und zweiten Leitungen jeweils darin einen Sensor (112, 124) aufweisen, um ein die Konzentration der durch die besagten Leitungen fließenden ersten und zweiten Dialysatlösung-Basis anzeigendes Signal zu erzeugen.

3. Einsatz (10) nach Anspruch 1 oder Anspruch 2, des Weiteren umfassend einen Sensor (116) in der dritten Leitung, um ein die Konzentration des dadurch fließenden Fluids anzeigendes Signal zu erzeugen.

4. Einsatz (10) nach einem der Ansprüche 1 bis 3, wobei die ersten und zweiten Fluidleitungen direkt in die Mischpumpenkammer (102) einspeisen.

5. Einsatz (10) nach Anspruch 3, des Weiteren umfassend:
ein Umsteuerungsventil (128, 130) in der dritten Leitung, flussabwärts zum darin enthaltenen Sensor (116), mit einer ersten Position, in der dort hindurch fließendes Fluid zum Dialysator geleitet wird, und mit einer zweiten Position, in der dort hindurch fließendes Fluid zu einem Ablass umgeleitet wird.

6. Ein Einsatz (10) nach einem der vorhergehenden Ansprüche, wobei die ersten und zweiten Fluidleitungen in eine Wasserleitung (104) unmittelbar flussaufwärts zur Kammer (102) der Mischpumpe einspeisen; oder wobei das erste Einlassventil (108) und das erste Auslassventil (108) und das zweite Einlassventil (122) und das zweite Auslassventil (122) Antisiphon-Rückschlagventile (200) umfassen, von denen jedes drei Anschlüsse und zwei Flussbahnen dort hindurch hat, eine, welche die Dialysatlösung-Basis-Bereitstellungsleitung mit der zugehörigen positiven Verdrängung und die zweite die zugehörige positive Verdrängungspumpe (110, 120, 212) mit der Mischpumpenkammer (102) verbindet.

7. Verfahren zum Kalibrieren einer Dialysat-Mischpumpe (102) zum Mischen eines Tri-Mix-Dialysats, umfassend eine Mischung der ersten Dialysat-Basislösung, zweiten Dialysat-Basislösung und Wasser in bestimmten Verhältnissen, umfassend die Schritte:
a) Vorhersagen des verlangten Volumens einer ersten Dialysatlösung-Basis, welche benötigt wird, ein Dialysat zu erzeugen, welches die verlangte Konzentration der ersten Dialysatlösung-Basis aufweist;
b) Bedienen einer ersten Dialysatlösung-Basis-Pumpe (110, 212), um das vorhergesagte Volumen der ersten Dialysatlösung-Basis einem Volumen von Wasser in einer Mischpumpe (102) hinzuzufügen und Mischen der ersten Dialysatlösung-Basis mit dem darin enthaltenen Wasser, um eine erste Mischung zu bilden;
c) Ausstoßen der ersten Mischung aus der Mischpumpe (102) und Messen eines Parameters, der die Konzentration der ersten Mischung anzeigt;
d) Modifizieren der Regulierungen der ersten Dialysatlösung-Basis-Pumpe (110, 212), um die Menge der zu der Mischpumpe (102) hinzugefügten ersten Dialysatlösung-Basis anzuheben oder zu vermindern, abhängig von dem gemessenen einen Parameter;
e) Vorhersagen des verlangten Volumens einer zweiten Dialysatlösung-Basis, welche benötigt wird, um ein eine verlangte Konzentration der zweiten Dialysatlösung-Basis aufweisendes Dialysat zu erzeugen;
f) Bedienen einer zweiten Dialysatlösung-Basis-Pumpe (120, 212), um das vorhergesagte Volumen der zweiten Dialysatlösung-Basis zu einem Volumen von Wasser in einer Mischpumpe (102) hinzuzufügen und Mischen der zweiten Dialysatlösung-Basis mit dem darin enthaltenen Wasser, um eine zweite Mischung zu bilden;
g) Ausstoßen der zweiten Mischung aus der Mischpumpe (102) und Messen eines die Konzentration der zweiten Mischung anzeigenden Parameters,
h) Modifizieren der Regulierungen der zweiten Dialysatlösung-Basis-Pumpe (120,212), um die Menge der zu der Mischpumpe (102) zugefügten zweiten Dialysatlösung-Basis anzuheben oder zu senken, abhängig von dem Parameter,
i) Bedienen der ersten (110, 212) und zweiten (120, 212) Dialysatlösung-Basis-Pumpen, um das modifizierte Volumen der ersten und zweiten Dialysatlösung-Basen zu einem Volumen von Wasser in der Mischpumpenkammer (102) hinzuzufügen, um einen verlangten Tri-Mix von erster Dialysat-Basislösung, zweiter Dialysat-Basislösung und Wasser zu erreichen.

8. Verfahren nach Anspruch 7, des Weiteren umfassend den Schritt einen die Konzentration der ersten Dialysatlösung-Basis anzeigenden Parameter zu messen und die Messung für die Vorhersage des verlangten Volumens der benötigten ersten Dialysatlösung-Basis zu verwenden.

9. Verfahren nach Anspruch 7 oder Anspruch 8, des Weiteren umfassend den Schritt einen die Konzentration der zweiten Dialysatlösung-Basis anzeigenden Parameter zu messen und die Messung für die Vorhersage des verlangten Volumens der benötigten zweiten Dialysatlösung-Basis zu verwenden.

10. Verfahren nach einem der Ansprüche 7 bis 9, wobei im Anschluss an Schritt h) das Verfahren des Weiteren die Schritte e) bis h) umfasst, bis die Konzentration der ersten Mischung der verlangten Konzentration der ersten Dialysatlösung-Basis gleich ist.

11. Verfahren nach Anspruch 10, des Weiteren umfassend folgenden Schritt:
sobald die verlangte Konzentration erreicht ist, einen Parameter aufzuzeichnen, welcher das Volumen der ersten Dialysatlösung-Basis anzeigt, welche zur Mischpumpe (102) hinzugefügt wurde, um eine erste Mischung mit der verlangten Konzentration zu erzeugen.

12. Verfahren nach einem der Ansprüche 7 bis 11, wobei im Anschluss an Schritt d) das Verfahren des Weiteren den Schritt umfasst, die Schritte a) bis d) zu wiederholen, bis die Konzentration der zweiten Mischung der verlangten Konzentration der zweiten Dialysatlösung-Basis gleich ist.

13. Verfahren nach Anspruch 12, des Weiteren umfassend folgenden Schritt:
sobald die verlangte Konzentration erreicht ist, einen Parameter aufzuzeichnen, welcher das Volumen der zweiten Dialysatlösung-Basis anzeigt, welche zur Mischpumpe (102) hinzugefügt wurde, um eine zweite Mischung mit der verlangten Konzentration zu erzeugen.

14. Verfahren nach Anspruch 7 des Weiteren umfassend: Messen eines Parameters, welcher die Konzentration des Tri-Mix anzeigt, um zu verifizieren, dass es die korrekte Konzentration aufweist.

15. Verfahren nach Anspruch 14 des Weiteren umfassend:
Wiederholen des Schrittes von Anspruch 7, sofern der gemessene Parameter anzeigt,
dass der Tri-Mix nicht die korrekte Konzentration aufweist.

16. Verfahren nach Anspruch 14 oder Anspruch 15 umfassend:
Anzeigen eines Parameters, welcher die Konzentration der ersten Dialysatlösung-Basis anzeigt.

17. Verfahren nach Anspruch 14, des Weiteren umfassend:
Wiederholen des Schritts von Anspruch 8, sofern der gemessene Parameter anzeigt,
dass die erste Dialysatlösung-Basis die Konzentration über einen spezifischen Bereich hinaus ändert.

18. Verfahren nach Anspruch 7, des Weiteren umfassend:
Anzeigen eines Parameters, der die Konzentration der zweiten Dialysatlösung-Basis anzeigt.

19. Verfahren nach Anspruch 18, des Weiteren umfassend:
Wiederholen des Schritts von Anspruch 8, sofern der gemessene Parameter anzeigt,
dass die zweite Dialysatlösung-Basis die Konzentration über einen spezifischen Bereich hinaus ändert.

20. Verfahren nach einem der Ansprüche 7 bis 19, des Weiteren umfassend:
Umleiten des Flusses des Dialysats zum Ablass, sofern die Mischung des Tri-Mix-Dialysats außerhalb ihrer spezifizierten Verhältnisse ist.

21. Verfahren nach einem der Ansprüche 7 bis 20, des Weiteren umfassend:
Umleiten des Flusses des Dialysats zu einem Dialysator (126), sofern die Mischung des Tri-Mix-Dialysats innerhalb ihrer spezifizierten Verhältnisse ist.

22. Einsatz nach einem der Ansprüche 1 bis 6, wobei der Einsatz (10) eine Blut-Flussbahn zum Transportieren eines Volumens von durch den Dialysator (126) zu behandelnden Blutes transportiert, oder wobei der Einsatz (10) ein Einwegeinsatz ist.

## Revendications

1. Cartouche (10) destinée à être utilisée dans une machine d'hémodialyse (1), ladite cartouche (10) comprenant :
un trajet d'écoulement de dialysat, comprenant un dialyseur, ledit trajet d'écoulement de dialysat étant destiné à fournir un écoulement de dialysat au sein dudit dialyseur ;
une pompe de mélange qui définit une chambre (102) ayant un volume variable entre un volume maximum et un volume minimum, et destinée à recevoir un volume prédéterminé d'une première base de solution de dialysat, d'une seconde base de solution de dialysat, et un volume d'eau ;
un premier conduit d'alimentation en base de solution de dialysat ayant une première pompe à déplacement positif (110, 212) ayant une première soupape d'admission (108) et une première soupape d'évacuation (108) associée à celle-ci ;
un second conduit d'alimentation en base de solution de dialysat ayant une seconde pompe à déplacement positif (120, 212) ayant une seconde soupape d'admission (122) et une seconde soupape d'évacuation (222) associée à celle-ci ;
un premier et un second conduits de fluide associés joignant lesdites première et seconde pompes à déplacement positif respectives (110, 120, 212) à ladite pompe de mélange (102) ; et
un troisième conduit de fluide destiné à relier l'évacuation de ladite pompe de mélange (102) à une admission de filtre de dialyseur (126).

2. Cartouche (10) selon la revendication 1, dans laquelle lesdits premier et second conduits contiennent chacun un capteur (112, 124) destiné à générer un signal qui indique la concentration desdites première et seconde bases de solution de dialysat qui s'écoulent dans lesdits conduits.

3. Cartouche (10) selon la revendication 1 ou 2, qui comprend en outre un capteur (116) dans ledit troisième conduit, destiné à générer un signal qui indique la concentration du fluide qui circule à l'intérieur.

4. Cartouche (10) selon l'une quelconque des revendications 1 à 3, dans laquelle lesdits premier et second conduits de fluide alimentent directement ladite chambre de pompe de mélange (102).

5. Cartouche (10) selon la revendication 3, qui comprend en outre :
une soupape directionnelle (128, 130) dans ledit troisième conduit, en avant dudit capteur (116), ayant une première position dans laquelle le fluide qui passe à l'intérieur est dirigé vers un dialyseur, et une seconde position dans laquelle le fluide qui passe à l'intérieur est dirigé vers une purge.

6. Cartouche (10) selon l'une quelconque des revendications précédentes, dans laquelle lesdits premier et second conduits de fluide alimentent un conduit d'eau (104) situé juste en amont de ladite chambre (102) de ladite pompe de mélange ; ou dans laquelle ladite première soupape d'admission (108) et ladite première soupape d'évacuation (108), et ladite seconde soupape d'admission (122) et ladite seconde soupape d'évacuation (122) comprennent un premier et un second clapets anti-retour et anti-siphon (200) ayant chacun trois orifices et deux trajets d'écoulement, l'un reliant ledit conduit d'alimentation en base de solution de dialysat à ladite pompe à déplacement positif associée, et l'autre reliant ladite pompe à déplacement positif associée (110, 120, 212) à ladite chambre de pompe de mélange (102).

7. Procédé d'étalonnage d'une pompe de mélange de dialysat (102) destinée à mélanger un dialysat trimix comprenant un mélange d'une première solution de base de dialysat, d'une seconde solution de base de dialysat et d'eau selon des rapports spécifiques, ledit procédé comprenant les étapes consistant à :
a) prédire le volume requis d'une première base de solution de dialysat nécessaire pour créer un dialysat ayant une concentration requise en première base de solution de dialysat ;
b) actionner une première pompe à base de solution de dialysat (110, 212) afin d'ajouter ledit volume prédit de première base de solution de dialysat à un volume d'eau dans une pompe de mélange (102), et mélanger ladite première base de solution de dialysat avec ladite eau afin de former un premier mélange ;
c) expulser ledit premier mélange de ladite pompe de mélange (102) et mesurer un paramètre qui indique la concentration dudit premier mélange ;
d) modifier les commandes de ladite première pompe à base de solution de dialysat (110, 212) afin d'augmenter ou de réduire la quantité de première base de solution de dialysat ajoutée à ladite pompe de mélange (102) selon ledit paramètre mesuré ;
e) prédire le volume requis d'une seconde base de solution de dialysat nécessaire pour créer un dialysat ayant une concentration requise en seconde base de solution de dialysat ;
f) actionner une seconde pompe à base de solution de dialysat (120, 212) afin d'ajouter ledit volume prédit de seconde base de solution de dialysat à un volume d'eau dans une pompe de mélange (102), et mélanger ladite seconde base de solution de dialysat avec ladite eau afin de former un second mélange ;
g) expulser ledit second mélange de ladite pompe de mélange (102) et mesurer un paramètre qui indique la concentration en second mélange ;
h) modifier les commandes de ladite seconde pompe à base de solution de dialysat (120, 212) afin d'augmenter ou de réduire la quantité de seconde base de solution de dialysat ajoutée à ladite pompe de mélange (102) selon ledit paramètre ;
i) actionner lesdites première (110, 212) et seconde (120, 212) pompes à base de solution de dialysat afin d'ajouter ledit volume modifié de première et de seconde bases de solution de dialysat à un volume d'eau dans ladite chambre de pompe de mélange (102) afin d'obtenir un trimix requis de ladite première solution de base de dialysat, de ladite seconde solution de base de dialysat, et d'eau.

8. Procédé selon la revendication 7, qui comprend en outre l'étape de mesure d'un paramètre qui indique la concentration en première base de solution de dialysat, et d'utilisation de ladite mesure pour prédire le volume requis de première base de solution de dialysat nécessaire.

9. Procédé selon la revendication 7 ou 8, qui comprend en outre l'étape de mesure d'un paramètre qui indique la concentration en seconde base de solution de dialysat, et d'utilisation de ladite mesure pour prédire le volume requis de seconde base de solution de dialysat nécessaire.

10. Procédé selon l'une quelconque des revendications 7 à 9, dans lequel, après l'étape h), ledit procédé comprend en outre les étapes e) à h) jusqu'à ce que la concentration en premier mélange soit égale à la concentration requise en première base de solution de dialysat.

11. Procédé selon la revendication 10, qui comprend en outre l'étape qui consiste à :
une fois que la concentration requise a été obtenue, enregistrer un paramètre qui indique le volume de la première base de solution de dialysat ajouté à ladite pompe de mélange (102) afin de créer un premier mélange ayant la concentration requise.

12. Procédé selon l'une quelconque des revendications 7 à 11, dans lequel, après l'étape d), ledit procédé comprend en outre l'étape qui consiste à répéter les étapes a) à d) jusqu'à ce que la concentration en second mélange soit égale à la concentration requise en seconde base de solution de dialysat.

13. Procédé selon la revendication 12, qui comprend en outre l'étape qui consiste à :
une fois que la concentration requise a été obtenue, enregistrer un paramètre qui indique le volume de seconde base de solution de dialysat ajouté à ladite pompe de mélange (102) afin de créer un second mélange ayant la concentration requise.

14. Procédé selon la revendication 7, qui comprend en outre :
la mesure d'un paramètre qui indique la concentration en trimix afin de vérifier qu'il possède bien la concentration requise.

15. Procédé selon la revendication 14, qui comprend en outre :
si ledit paramètre mesuré indique que ledit trimix ne possède pas la concentration requise, la répétition de l'étape de la revendication 7.

16. Procédé selon la revendication 14 ou 15, qui comprend :
le suivi d'un paramètre qui indique la concentration en première base de solution de dialysat.

17. Procédé selon la revendication 14, qui comprend en outre :
si ledit paramètre mesuré indique que ladite première base de solution de dialysat change de concentration au-delà d'une plage spécifiée, la répétition des étapes de la revendication 8.

18. Procédé selon la revendication 7, qui comprend en outre :
le suivi d'un paramètre qui indique la concentration en seconde base de solution de dialysat.

19. Procédé selon la revendication 18, qui comprend en outre :
si ledit paramètre mesuré indique que ladite seconde base de solution de dialysat change de concentration au-delà d'une plage spécifiée, la répétition des étapes de la revendication 8.

20. Procédé selon l'une quelconque des revendications 7 à 19, qui comprend en outre :
si ledit mélange dudit dialysat trimix se trouve en-dehors de ses rapports spécifiés, l'orientation de l'écoulement de dialysat vers ladite purge.

21. Procédé selon l'une quelconque des revendications 7 à 20, qui comprend en outre :
si ledit mélange dudit dialysat trimix se trouve dans les limites de ses rapports spécifiés, l'orientation de l'écoulement de dialysat vers un dialyseur (126).

22. Cartouche selon l'une quelconque des revendications 1 à 6, où la cartouche (10) comprend un trajet d'écoulement du sang destiné à acheminer un volume de sang à traiter par un dialyseur (126), ou qui est jetable.
